# FASCICULE DE BREVET EUROPEEN

(11) **EP 2 227 204 B1**
(45) Date de publication et mention de la délivrance du brevet: **19.12.2012**
(21) Numéro de dépôt: 09704243.6
(22) Date de dépôt: 07.01.2009
(51) Int. Cl.: A61F 13/08, D04B 9/52

(54) **ARTICLE TEXTILE TUBULAIRE DE COMPRESSION**
RÖHRENFÖRMIG KOMPRIMIERTER BEKLEIDUNGSARTIKEL
TUBULAR COMPRESSION TEXTILE ARTICLE

(30) Priorité: 08.01.2008 FR 0850086
(43) Date de publication de la demande: 15.09.2010
(73) Titulaire: THUASNE, 92300 Levallois-Perret (FR)
(72) Inventeur: CONVERT, Reynald, F-42800 Saint Martin La Plaine (FR); CATTIAUX, Gérard, F-42480 La Fouillouse (FR)
(74) Mandataire: Balesta, Pierre
(86) Numéro de dépôt international: PCT/FR2009/050011
(87) Numéro de publication internationale: WO 2009/092932

(56) Documents cités:
- FR-A- 2 432 867
- FR-A- 2 843 877

## Description

La présente invention est dans le domaine des articles textiles tubulaires de compression, et plus particulièrement des articles comprenant un premier et un second éléments tubulaires de compression, destinés à être superposés lors de l'enfilage de l'article.

Les articles de compression, du type bas/chaussette ou manchon, sont utilisés pour prévenir ou soigner les problèmes de circulation veineuse notamment au niveau du membre inférieur, ainsi que les pathologies liées aux dysfonctionnements du système lymphatique et réduire les oedèmes. Les troubles veineux peuvent avoir plusieurs origines, parmi celles-ci on trouve notamment : une rigidification de la paroi veineuse, une altération des valvules ou encore une augmentation du diamètre des veines.

La pression locale exercée sur un membre par un article de compression est fonction notamment des caractéristiques de force-allongement dudit article.

La pression exercée sur un membre se calcule par la loi de Laplace suivante : P [Pa ou mmHg] = ( T [N] X n / (L [m] X R [m]).

P représente la pression exercée en un point donné du membre considéré.

T est la tension, exprimée en Newtons, exercée par le dit article lorsqu'il est enfilé sur le membre inférieur ou supérieur.

R est le rayon de courbure au point considéré du membre inférieur ou supérieur.

Plus la déficience du système veineux est importante, plus le sang présente des difficultés à refluer depuis la cheville ou le poignet vers le coeur, et plus la pression à exercer au niveau de la cheville ou du poignet est importante.

A titre d'exemple dans le système français, les niveaux de compression peuvent être répartis ainsi :

| | | | |
|---|---|---|---|
| Classe I : | 13 à 20 hPa | Classe II : | 20,1 à 27 hPa |
| Classe III : | 27,1 hPa à 48 hPa | Classe IV : | plus de 48 hPa |

Les articles de compression, notamment de classe élevée, sont difficiles à enfiler par le patient, notamment lorsque celui-ci souffre d'une mobilité réduite. Une des solutions proposée actuellement est de superposer un premier élément de compression et un second élément de compression ayant individuellement un effet de compression inférieur à la compression finale visée mais dont les effets de compression s'additionnant permettent d'atteindre celle-ci.

Dans FR 2.432.867 au nom du demandeur, l'article de compression pour une jambe se présente sous la forme d'un tube tricoté unique ayant sur sa longueur deux zones pour recevoir le talon. Le tube tricoté est ouvert à ses deux extrémités. L'usager enfile alors le tube à partir de l'une des extrémités telle une chaussette en plaçant correctement son talon dans la première zone correspondante, retourne le tube en sorte de placer son talon dans la seconde zone correspondante puis enfile le tube retourné tel un bas.

Ce type d'article de compression présente l'avantage que le bas et la chaussette sont formés d'un seul tenant. Néanmoins au porter, le premier élément de compression correspondant au bas a tendance à glisser sur le second élément correspondant à la chaussette. Il se forme alors des plis, en particulier au niveau du pied, très gênant pour le patient. De plus, cet article n'est valable que pour les pointes de pied ouvertes. Enfin, il est difficile à positionner correctement puisque l'usager n'a aucune aide pour repérer l'extrémité distale dudit article correspondant au retournement du premier élément sur le second élément de compression. En effet, si les éléments de bas et de chaussette ne se superposent pas correctement, la compression exercée et sa dégressivité sont altérées.

L'article textile tubulaire de compression selon la présente invention, notamment pour un membre supérieur ou un membre inférieur, comprenant un premier et un second éléments tubulaires de compression, destinés à être superposés lors de l'enfilage de l'article, a pour but de résoudre tout ou partie des problèmes précités en ce que lesdits premier et second éléments sont reliés l'un à l'autre selon une zone circulaire de jonction de laquelle se prolonge un troisième élément qui est destiné à former l'extrémité distale dudit article lorsque ledit article est enfilé.

Ledit troisième élément est ainsi agencé en sorte de former l'extrémité distale dudit article textile tubulaire en fonctionnement.

Le troisième élément, de préférence tubulaire, étant parfaitement distinct des premier et second éléments tubulaires, a pour fonction d'empêcher le premier élément tubulaire de glisser sur le second élément tubulaire au-delà de la zone circulaire de jonction et donc de faciliter l'enfilage dudit article sans formation de plis. Les premier et second éléments tubulaires sont ainsi parfaitement superposés. Une simple zone circulaire de jonction serait insuffisante pour empêcher totalement le premier élément tubulaire de glisser sur le second élément tubulaire ou inversement.

De préférence, le troisième élément est également destiné à être enfilé sur le membre à soigner.

En fonction de la confection du troisième élément, ledit article pour un membre inférieur peut être à pointe de pied fermée ou ouverte.

La zone circulaire de jonction solidarise les premier et second éléments tubulaires de compression selon tout ou partie de leurs circonférences de sorte qu'il ne s'agit pas d'une zone sur laquelle le pied par exemple vient en butée. Une fois le premier élément de compression sur le membre, le second élément étant joint au premier élément selon ladite zone de jonction, est déjà positionné par rapport au premier élément ce qui facilite une superposition correcte. De plus, la zone de jonction forme une zone de blocage du premier élément par rapport au second élément empêchant ainsi le premier élément de glisser sur le second élément de compression, notamment dans le cas d'une pointe de pied ouverte.

De préférence, deux régions de talon sont ménagées respectivement sur les premier et second éléments de compression en sorte d'améliorer la facilité du positionnement de chaque élément et le confort de l'article.

Dans une variante de réalisation, le troisième élément étant tubulaire, ses extrémités avant et arrière sont reliées à la zone de jonction en sorte de former un repli. Le troisième élément est ainsi formé de deux couches textiles tubulaires superposées qui sont ainsi agencées compte tenu du procédé de fabrication permettant la réalisation dudit article par tricotage continu des premier, second et troisième éléments tubulaires.

La partie du troisième élément qui est à l'opposé de la zone de jonction n'étant pas fermée, forme une extrémité distale de l'article ouverte pour le pied ou le poignet par exemple.

Dans une variante de réalisation, la partie du troisième élément, qui est à l'opposé de la zone de jonction, est fermée sur elle-même en sorte de former, après enfilage, l'extrémité distale dudit article.

Ladite extrémité distale correspond notamment à la pointe du pied fermée lorsque ledit article est pour un membre inférieur.

Dans une variante de réalisation, le troisième élément a, au repos, une longueur comprise dans l'intervalle allant de [5 mm ; 200 mm], de préférence [5 mm ; 70 mm].

Lorsque l'extrémité distale formée dans le troisième élément est ouverte, le troisième élément a, de préférence, une longueur comprise dans l'intervalle [5 mm ; 30 mm].

Lorsque l'extrémité distale formée dans le troisième élément est fermée, formant alors la pointe du pied fermée, le troisième élément a de préférence une longueur comprise dans l'intervalle [30 mm ; 70 mm]

Dans une variante de réalisation, le troisième élément est sans effet de compression.

On entend par « sans effet de compression » que l'effet de compression exercé est inférieur à 13 hPa, correspondant à la limite inférieure de la classe I.

Il est ainsi plus aisé pour l'usager de retourner le second élément de compression sur le premier élément de compression. De plus, le troisième élément ayant une longueur réduite par rapport aux premier et second éléments, il doit être confortable et ne pas agir tel un garrot sur le membre à traiter. Cette disposition favorise également la confection du troisième élément pour former notamment une pointe de pied fermée.

Dans une variante de réalisation, la zone de jonction est cousue, soudée par ultrasons ou hautes fréquences, ou tricotée selon tout ou partie de la circonférence des premier et second éléments tubulaires.

Dans une variante de réalisation, ledit article est formé d'un seul tenant.

Avantageusement, les premier, second et troisièmes éléments sont formés à partir d'un même tube, de préférence tricoté, ce qui évite les approximations dues au montage, notamment par confection lorsque l'on rapporte une chaussette sur un bas par exemple et que ces derniers ont été tricotés séparément. Les défauts lors du montage peuvent nuire à la régularité de la dégressivité de la compression exercée sur le membre.

Dans une variante de réalisation, ledit article étant pour un membre inférieur, chaque premier élément et second élément est du type bas ou chaussette.

Les premier et second éléments de compression peuvent être du type: bas/bas, chaussette/chaussette ou bas/chaussette.

La présente invention a pour objet, selon un deuxième aspect, l'utilisation d'un article de compression selon l'une des variantes de réalisation précédentes pour la compression d'un membre supérieur.

Les premier et second éléments de compression peuvent être des manchons. Le premier élément recouvrant par exemple l'avant-bras et le second élément recouvrant, en plus de l'avant-bras, le bras.

La présente invention a également pour objet, selon un troisième aspect, un procédé de fabrication d'un article textile de compression comprenant les étapes suivantes :
a) tricoter en continu un premier élément tubulaire de compression, un élément intermédiaire tubulaire puis un second élément tubulaire de compression, lesdits premier et second éléments étant destinés à être superposés lors de l'enfilage dudit l'article, et de manière caractéristique :
b) relier lesdits premier et second éléments selon une zone circulaire de jonction soit par report de mailles lors du tricotage au cours de l'étape a) soit après l'étape a) en rentrant le second élément à l'intérieur du premier élément et en réalisant ladite zone de jonction par couture, soudure par ultra-sons ou haute fréquences ou collage en sorte que ledit élément intermédiaire se projette de ladite zone circulaire et que ses extrémités avant et arrière soient reliées à ladite zone de jonction en sorte de former un repli.

La technique de report de mailles par tricotage est connue de l'homme du métier. Le report de mailles peut être opéré d'une aiguille sur l'autre, d'une fonture sur l'autre ou d'une fonture sur un cylindre et inversement. Dans le procédé selon la présente invention le report de mailles est, de préférence, vertical. Ce report de mailles peut être réalisé notamment sur les métiers à tricoter suivants : métier circulaire équipé d'un cylindre et d'un plateau à aiguilles, ou de deux cylindres.

De préférence, le report de mailles vertical est réalisé dans l'étape a) sur un métier circulaire entre le cylindre et le plateau. Les mailles délimitant l'extrémité avant du repli sont reportées lors du tricotage sur les mailles délimitant l'extrémité arrière du repli.

L'article selon la présente invention peut être également tricoté sur un métier rectiligne à double fonture, auquel cas la formation du repli se fait lors de la confection.

De préférence, le repli est formé par report de mailles lors du tricotage en continu. Cette disposition évite les risques d'approximations dues au montage entre les premier et second éléments de compression, et supprime une étape ultérieure de confection.

Dans une variante de réalisation, ledit procédé comprend une étape c) au cours de laquelle on ferme, notamment par couture, la partie de l'élément intermédiaire replié ou repli qui est à l'opposé de la zone de jonction en sorte de former, après enfilage, l'extrémité distale dudit article, notamment la pointe du pied.

Les caractéristiques de l'élément intermédiaire tubulaire, telle que sa longueur ou son allongement, dépendent des fils sélectionnés et des paramètres de tricotage.

Dans une variante de réalisation, le métier à tricoter employé dans l'étape a) est un métier circulaire, avec 4 chutes, de préférence en jauge 20.

La présente invention sera mieux comprise à la lecture de deux exemples de réalisation, cités à titre non limitatif, et illustrés dans les figures suivantes, annexées à la présente, et dans lesquelles :
- la figure 1 est une représentation d'un premier exemple d'article selon la présente invention après l'étape de tricotage,
- la figure 2 est une représentation du premier exemple d'article représenté à la figure 1 après l'étape de formation du repli et enfilage,
- la figure 3 est une représentation d'un second exemple d'article selon la présente invention après l'étape de tricotage,
- les figures 4 et 5 illustrent la formation de la pointe de pied fermée du second exemple de l'article représenté à la figure 3,
- les figures 6, 7 et 8 illustrent des variantes d'armure de tricotage des articles selon la présente invention.

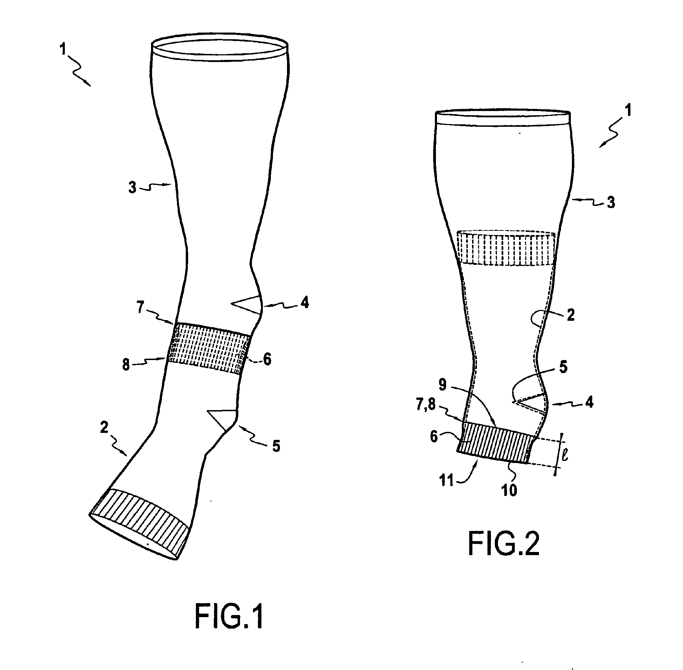

L'article textile tubulaire de compression 1, représenté aux figures 1 et 2, est destiné à un membre inférieur. Ledit article 1 comprend un premier 2 et un second 3 éléments tubulaires de compression destinés à être superposés lors de l'enfilage de l'article 1. Les premier 2 et second 3 éléments tubulaires correspondent respectivement à une chaussette et à un bas. Le premier 2 et le second 3 élément tubulaires comprennent respectivement une première 5 et une seconde 4 zones de talon destinées à recevoir le talon. L'article 1 représenté à la figure 1 est obtenu suite à l'étape de tricotage en continu et est d'un seul tenant. Un élément intermédiaire tubulaire 6 sépare et lie les premier 2 et second 3 éléments tubulaires. Les première 5 et seconde 4 zones de talon sont disposées de part et d'autre dudit élément intermédiaire 6. Lors de la finalisation de l'article 1, le premier élément 2 est rentré dans le second élément 3 puis les extrémités avant 7 et arrière 8 de l'élément intermédiaire 6 sont reliées selon une zone circulaire de jonction 9 en sorte que l'élément intermédiaire 6 forme un repli 10. Le premier élément 2 et le second élément 3 sont ainsi reliés l'un à l'autre selon la zone circulaire de jonction 9, c'est à dire selon tout le pourtour de leurs circonférences. L'élément intermédiaire 6 replié ou repli 10, également appelé troisième élément tubulaire, prolonge la zone circulaire de jonction 9. La partie 11 du repli 10 qui est à l'opposé de la zone de jonction circulaire 9 est ouverte. L'article 1 de compression est donc employé pour la compression de la jambe avec une pointe de pied ouverte correspondant à ladite partie 11 du repli 10. Le repli 10 est donc formé de la superposition de deux couches d'élément intermédiaire 6.

La zone circulaire de jonction 9 est dans cet exemple précis réalisée par couture ou par tout autre moyen connu de l'état de la technique, telle que par soudure ultra-sons ou hautes fréquences.

L'élément intermédiaire 6 et donc le repli 10 sont sans effet de compression, ou si effet de compression il y a un, ce dernier est inférieur à 13 hPa correspondant à la limite inférieure de la classe I. Le repli 10 est ainsi facile à positionner par le patient, notamment en cas de mobilité réduite.

Le troisième élément ou repli 10 a une longueur ℓ comprise dans l'intervalle [5 mm ; 30 mm], et dans cet exemple précis une longueur ℓ au repos (sans aucune contrainte mécanique) de l'ordre de 10 mm.

Les première 5 et seconde 4 zones de talon sont de préférence obtenues lors du tricotage en continu par diminution et augmentation. Cette opération de façonnage, bien connue de l'homme du métier, est directement réalisée sur le métier à tricoter suivant un schéma de mailles déterminé qui diminue ou augmente le nombre d'aiguilles en action pour réaliser une mise en forme particulière. Cette opération peut être réalisée sur autant de rangées de mailles que nécessaire.

Le procédé de fabrication préféré, mais non représenté pour des raisons de simplification, consiste à tricoter en continu le premier élément tubulaire de compression 2, l'élément intermédiaire tubulaire 6 puis à reporter verticalement et lier les mailles de l'extrémité avant 7 dudit élément intermédiaire 6 sur les mailles de l'extrémité arrière 8 dudit élément intermédiaire 6, et enfin tricoter le second élément tubulaire de compression 3. L'article 1 est de préférence tricoté sur un métier circulaire, du type cylindre-plateau, avec 4 chutes, en jauge 20 par exemple. Des changements de fils peuvent être effectués à tout moment entre les première 5 et seconde 4 zones de talon, les premier 2 et second 3 éléments tubulaires, ainsi que l'élément intermédiaire 6 selon les propriétés souhaitées.

A titre d'exemple, le premier élément 2 est tricoté avec un fil, dit de maille ou de fond, en élasthanne de 22 dtex guipé avec un fil en PA (polyamide) de 22 dtex et un fil en PA de 13 dtex, et avec un fil, dit de trame, d'élasthanne de 285 dtex guipé avec deux fils en PA de 22 dtex chacun. La première zone 5 de talon et sensiblement la moitié de l'élément intermédiaire 6 se prolongeant du premier élément 2 sont tricotés avec un fil d'élasthanne de 156 dtex guipé avec deux fils en PA de 44 dtex chacun. La moitié restante de l'élément intermédiaire 6, se prolongeant vers le second élément 3, et la seconde zone 4 de talon sont tricotés avec un fil d'élasthanne de 22 dtex guipé avec un fil en polyamide de 44 dtex et un fil de coton de 2/200 Nm. Le second élément de compression 3 est tricoté avec un fil, dit de maille ou de fond, en élasthanne de 22 dtex guipé avec un fil en polyamide de 44 dtex et un fil de coton de 2/200 Nm, et avec un fil, dit de trame, en élasthanne de 330 dtex guipé avec un fil en PA de 22 dtex et un fil de coton de 160 Nm. Dans cet exemple précis, le fil de tricotage pour l'élément intermédiaire 6 est changé vers la moitié dudit élément intermédiaire 6.

Les caractéristiques de tricotage, notamment la LFA (longueur de fil absorbé), sont également déterminées en sorte d'obtenir à titre d'exemple un effet de compression sur la cheville de 18 hPa pour le premier élément de compression 2 et de 19 hPa pour le second élément de compression 3. L'effet de compression dudit article 1, les premier 2 et second 3 éléments étant superposés sur la cheville, est alors de 37 hPa. Ainsi, un premier 2 et second 3 éléments de compression de classe I superposés permettent d'obtenir un niveau de compression de classe III.

Pour l'enfilage de l'article 1, l'usager dispose tout d'abord le premier élément de compression 2 formant une chaussette sur sa jambe de sorte de disposer son talon dans la première zone de talon 5. Le repli 10 est alors parfaitement positionné sur son pied. Le second élément 3 étant relié au premier élément 2 dans la zone de jonction tubulaire 9, l'usager n'a plus qu'à enfiler le second élément 3 sur le premier élément 2, jusqu'à placement de son talon dans la seconde zone de talon 4. La zone de jonction 9, en combinaison avec l'élément intermédiaire 6 formant un troisième élément tubulaire, permet naturellement de repérer l'extrémité distale de l'article 1 et donc l'emplacement selon lequel l'usager doit retourner le second élément 3 sur le premier élément 2. Le premier élément tubulaire 2 et le second élément tubulaire 3 ne peuvent glisser l'un sur l'autre grâce à la présence de l'élément intermédiaire 6 se projetant de la zone circulaire de jonction 9. La superposition des premier 2 et second 3 éléments est donc parfaite lors de la pose dudit article 1 en sorte d'obtenir une addition des effets de compression homogène et une dégressivité de la compression régulière de la cheville vers le haut de la jambe. De plus, le repli 10, étant de préférence sans effet de compression, allié aux éléments 4 et 5, empêche la tendance qu'aurait le premier élément 2 à glisser sur le second élément 3 notamment dans le cas d'une pointe de pied ouverte 11, ce qui améliore le confort du patient. De préférence, la confection de la zone circulaire 9 est déterminée en sorte que lorsque l'article 1 est porté, tel que représenté à la figure 2, le repli 10 débouche sur l'extérieur et ne soit pas coincé entre les premier 2 et second 3 éléments tubulaires de compression.

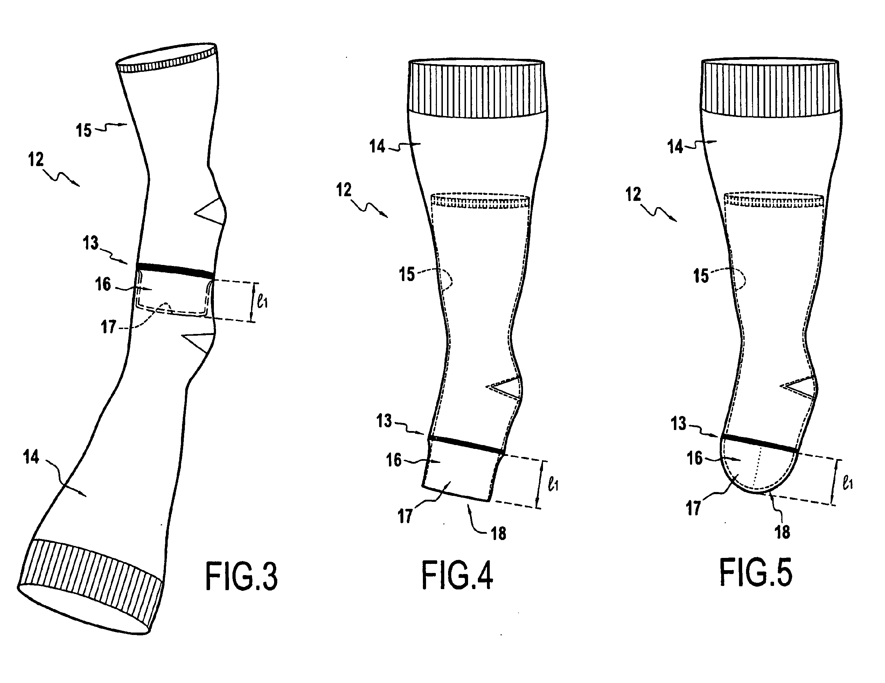

Le second exemple d'article 12 selon la présente invention, et illustrés dans les figures 3 à 6, diffère notamment du premier exemple d'article 1 en ce que la pointe de pied est fermée.

L'article 12 représenté à la figure 4 est obtenu suite à l'étape de tricotage en continu. La zone circulaire de jonction 13 a été formée par report de mailles et relie un premier élément de compression 14, dans cet exemple précis une bas, avec un second élément de compression 15, ici une chaussette. L'élément intermédiaire 16 ou troisième élément prolonge la zone circulaire de jonction 13. Les figures 4 et 5 illustrent la formation de la pointe du pied. Lors du tricotage, l'élément intermédiaire 16 a été façonné de sorte que sa longueur ℓ1 soit de l'ordre une fois replié compris dans l'intervalle [30 mm ; 200 mm], et dans cet exemple précis de l'ordre de 75 mm. Le premier élément 14 est superposé au second élément 15 tel que représenté à la figure 4 pour dégager le repli 17. La partie du repli 18 à l'opposé de la zone de jonction 13 est fermée, de préférence par couture, en sorte de former une pointe de pied fermée sur laquelle vient en butée le pied lors de l'enfilage.

Tel que pour le premier exemple d'article 1, l'usager enfile le second élément 15 puis superpose le premier élément 14 à partir de la zone de jonction circulaire 13.

Dans ce second exemple 12, l'élément intermédiaire 16 replié formant le repli 17 est également sans effet de compression, ce qui améliore le confort du patient puisqu'il n'est pas nécessaire d'exercer dans cette région du corps un effet de compression.

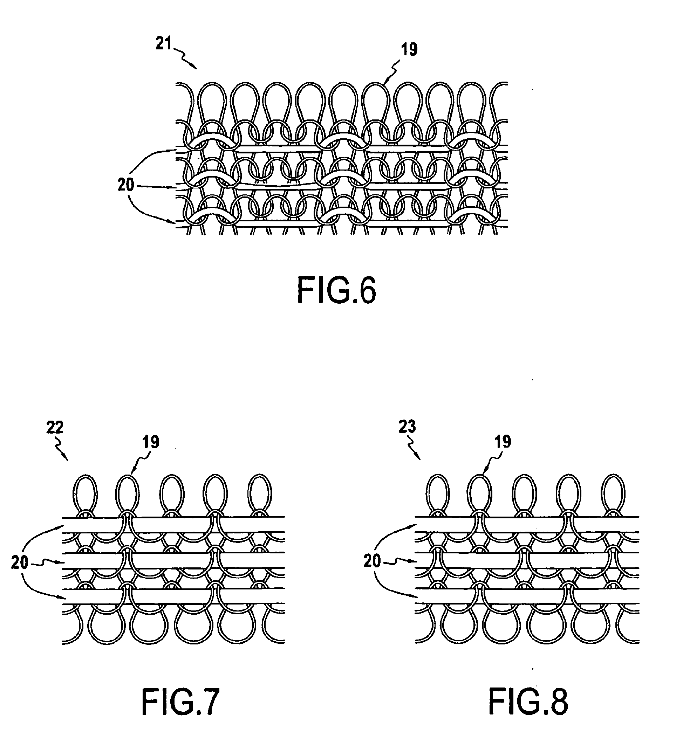

Les figures 6 à 8 illustrent des variantes d'armure de tricotage pour le tricotage indifféremment des articles 1 ou 15.

Les mêmes références sont employées pour désigner les fils dit de maille ou de fond 19 et les fils dits de trame 20 sur les armures 21,22 et 23. Sur l'armure 22, les fils dits de trame ne sont pas en quinconce ce qui signifie qu'ils sont pris de la même façon sur chaque rangée par les pieds de mailles de la rangée supérieure. Sur l'armure 23, les fils dits de trame sont en quinconce puisqu'ils ne sont pas pris de la même façon par les pieds de maille entre deux rangées.

Il est possible de placer lors de l'enfilage des articles de compression 1 et 12 des accessoires tels que des plaques, des plots ou coussins en mousse, destinés à renforcer la compression dans les régions creuses du corps ou protéger certaines régions dans lesquelles la pression trop forte risque de blesser (exemple : tendon d'Achille, crête tibiale, ...).

De tels accessoires peuvent être disposés lors de l'enfilage par l'usager entre le premier élément de compression et le second élément de compression.

## Revendications

1. Article textile tubulaire (1,12) de compression, notamment pour un membre supérieur ou un membre inférieur, comprenant un premier (2,14) et un second (3,15) éléments tubulaires de compression, destinés à être superposés lors de l'enfilage de l'article (1,12) **caractérisé en ce que** lesdits premier (2,14) et second (3,15) éléments sont reliés l'un à l'autre selon une zone circulaire de jonction (9,13) de laquelle se prolonge un troisième élément (6,16) qui est destiné à former l'extrémité distale dudit article lorsque ledit article est enfilé.

2. Article textile (1,12) selon la revendication 1, **caractérisé en ce que** le troisième élément (6,16) étant tubulaire, ses extrémités avant (7) et arrière (8) sont reliées à la zone de jonction (9,13) en sorte de former un repli (10,17).

3. Article textile (12) selon l'une ou l'autre des revendications 1 et 2, **caractérisé en ce que** la partie (18) du troisième élément (16) qui est à l'opposé de la zone de jonction (13) est fermée sur elle-même en sorte de former, après enfilage, l'extrémité distale dudit article.

4. Article textile (1,12) selon l'une quelconque des revendications 1 à 3, **caractérisé en ce que** le troisième élément (6,16) a, au repos, une longueur (ℓ,ℓ1) comprise dans l'intervalle allant de [5 mm ; 200 mm], de préférence [5 mm ; 70 mm].

5. Article textile (1,12) selon l'une quelconque des revendications 1 à 4, **caractérisé en ce que** le troisième élément (6,16) est sans effet de compression.

6. Article textile (1,12) selon l'une quelconque des revendications 1 à 5, **caractérisé en ce que** la zone de jonction (9,13) est cousue, soudée par ultrasons ou hautes fréquences, ou tricotée selon tout ou partie de la circonférence des premier (2,14) et second (3,15) éléments.

7. Article textile (1,12) selon l'une quelconque des revendications 1 à 5, **caractérisé en ce qu'**il est formé d'un seul tenant.

8. Article textile (1,12) de compression pour un membre inférieur selon l'une quelconque des revendications 1 à 7, **caractérisé en ce que** chaque premier élément (2,14) et second élément (3,15) sont du type bas ou chaussette.

9. Article textile de compression pour un membre supérieur, selon l'une quelconque des revendications 1 à 7, **caractérisé en ce que** les premier et second éléments sont des manchons et **en ce que** le premier élément recouvre l'avant-bras et le second élément recouvre l'avant-bras et le bras.

10. Procédé de fabrication d'un article textile (1,12) de compression comprenant l'étape suivante :
a) tricoter en continue un premier élément tubulaire de compression (2,14), un élément intermédiaire tubulaire (6,16) puis un second élément tubulaire de compression (3,15), lesdits premier (2,14) et second (3,15) éléments étant destinés à être superposés lors de l'enfilage dudit l'article (1,12),
**caractérisé en ce qu'**il comprend l'étape suivante:
b) relier lesdits premier (2,14) et second (3,15) éléments selon une zone circulaire de jonction (9,13) soit par report de mailles lors du tricotage au cours de l'étape a) soit après l'étape a) en rentrant le second élément (3,15) à l'intérieur du premier élément (2,14) et en réalisant ladite zone de jonction (9,13) par couture, soudure par ultra-sons ou haute fréquences ou collage en sorte que ledit élément intermédiaire (6,16) se projette de ladite zone circulaire (9,13) et que ses extrémités avant (7) et arrière (8) soient reliées à ladite zone de jonction (9,13) en sorte de forme un repli (10,17).

11. Procédé de fabrication selon la revendication 10, **caractérisé en ce qu'**il comprend une étape c) au cours de laquelle on ferme, notamment par couture, la partie (18) du repli (17) qui est à l'opposé de la zone de jonction (13) en sorte de former, après enfilage, l'extrémité distale dudit article, notamment la pointe du pied.

12. Procédé de fabrication selon l'une ou l'autre des revendications 10 et 11 **caractérisé en ce que** l'étape de tricotage en continu est réalisée sur un métier circulaire avec 4 chutes, de préférence en jauge 20.

## Claims

1. A tubular compression textile article (1, 12), especially for an upper member or a lower member, comprising a first (2, 14) and a second (3, 15) tubular compression element, designed to be superposed during fitting of the article (1, 12), **characterised in that** said first (2, 14) and second (3, 15) elements are connected to each other according to a circular junction area (9, 13) from which a third element (6, 16) extends which is designed to form the distal end of said article when said article is fitted.

2. The textile article (1, 12) as claimed in Claim 1, **characterised in that** since the third element (6, 16) is tubular, its front (7) and rear (8) ends are connected to the junction area (9, 13) so as to form a fold (10, 17).

3. The textile article (12) as claimed in either of Claims 1 and 2, **characterised in that** the part (18) of the third element (16) which is opposite the junction area (13) is closed on itself so as to form the distal end of said article after fitting.

4. The textile article (1, 12) as claimed in any one of Claims 1 to 3, **characterised in that** when at rest the third element (6, 16) has a length (ℓ, ℓ1) ranging from [5 mm ; 200 mm], preferably [5 mm ; 70 mm].

5. The textile article (1, 12) as claimed in any one of Claims 1 to 4, **characterised in that** the third element (6, 16) has no compression effect.

6. The textile article (1, 12) as claimed in any one of Claims 1 to 5, **characterised in that** the junction area (9, 13) is stitched, welded by ultrasound or high-frequency, or knitted according to all or part of the circumference of the first (2, 14) and second (3, 15) elements.

7. The textile article (1, 12) as claimed in any one of Claims 1 to 5, **characterised in that** it is formed in a single piece.

8. The compression textile article (1, 12) for a lower member as claimed in any one of Claims 1 to 7, **characterised in that** each first element (2, 14) and second element (3, 15) are of the hosiery or sock type.

9. The compression textile article for an upper member, as claimed in any one of Claims 1 to 7, **characterised in that** the first and second elements are sleeves and **in that** the first element covers the forearm and the second element covers the forearm and the arms.

10. A manufacturing process for a compression textile article (1, 12) comprising the following step:
a) continuous knitting of a first tubular compression element (2, 14), an intermediate tubular element (6, 16) then a second tubular compression element (3, 15), said first (2, 14) and second (3, 15) elements being designed to be superposed during fitting of said article (1, 12),
**characterised in that** it comprises the following step:
b) connecting said first (2, 14) and second (3, 15) elements according to a circular junction area (9, 13) either by extension of mesh during knitting during step a) or after step a) by returning the second element (3, 15) inside the first element (2, 14) and by making said junction area (9, 13) by stitching, welding by ultrasound or high-frequency or adhesion such that said intermediate element (6, 16) projects from said circular area (9, 13) and its front (7) and rear (8) ends are connected to said junction area (9, 13) so as to form a fold (10, 17).

11. The manufacturing process as claimed in Claim 10, **characterised in that** it comprises a step c) during which the part (18) of the fold (17) which is opposite the junction area (13) is closed, especially by stitching, so as to form the distal end of said article, especially the point of the foot after fitting.

12. The manufacturing process as claimed in either of Claims 10 and 11, **characterised in that** the continuous knitting step is performed on a circular loom with 4 feeds, preferably gauge 20.

## Patentansprüche

1. Röhrenförmiger Kompressionstextilartikel (1, 12), insbesondere für eine obere Gliedmaße oder eine untere Gliedmaße, umfassend ein erstes (2, 14) und ein zweites (3, 15) röhrenförmiges Kompressionselement, die dazu bestimmt sind, beim Überziehen des Artikels (1, 12) übereinander gelegt zu werden, **dadurch gekennzeichnet, daß** das erste (2, 14) und das zweite (3, 15) Element entlang einem kreisförmigen Verbindungsbereich (9, 13) miteinander verbunden sind, von dem aus sich ein drittes Element (6, 16) fortsetzt, das dazu bestimmt ist, das distale Ende des Artikels zu bilden, wenn der Artikel übergezogen ist.

2. Textilartikel (1, 12) nach Anspruch 1, **dadurch gekennzeichnet, daß**, da das dritte Element (6, 16) röhrenförmig ist, sein vorderes (7) und sein hinteres (8) Ende mit dem Verbindungsbereich (9, 13) verbunden sind, um einen Umschlag (10, 17) zu bilden.

3. Textilartikel (12) nach dem einen oder anderen der Ansprüche 1 und 2, **dadurch gekennzeichnet, daß** der Teil (18) des dritten Elements (16), der von dem Verbindungsbereich (13) abgewandt ist, in sich geschlossen ist, um nach dem Überziehen das distale Ende des Artikels zu bilden.

4. Textilartikel (1, 12) nach einem der Ansprüche 1 bis 3, **dadurch gekennzeichnet, daß** das dritte Element (6, 16) in Ruhe eine Länge (I, I1) im Bereich von [5 mm; 200 mm], vorzugsweise [5 mm; 70 mm] aufweist.

5. Textilartikel (1, 12) nach einem der Ansprüche 1 bis 4, **dadurch gekennzeichnet, daß** das dritte Element (6, 16) keine Kompressionswirkung aufweist.

6. Textilartikel (1, 12) nach einem der Ansprüche 1 bis 5, **dadurch gekennzeichnet, daß** der Verbindungsbereich (9, 13) entlang dem gesamten Umfang des ersten (2, 14) und des zweiten (3, 15) Elements oder einem Teil dessen angenäht, mittels Ultraschall oder Hochfrequenz angeschweißt oder angestrickt ist.

7. Textilartikel (1, 12) nach einem der Ansprüche 1 bis 5, **dadurch gekennzeichnet, daß** er einstückig ausgebildet ist.

8. Kompressionstextilartikel (1, 12) für eine untere Gliedmaße, nach einem der Ansprüche 1 bis 7, **dadurch gekennzeichnet, daß** jedes erste Element (2, 14) und zweite Element (3, 15) vom Typ Strumpf oder Socken sind.

9. Kompressionstextilartikel für eine obere Gliedmaße, nach einem der Ansprüche 1 bis 7, **dadurch gekennzeichnet, daß** das erste und das zweite Element Manschetten sind und daß das erste Element den Unterarm und das zweite Element den Unterarm und den Oberarm bedeckt.

10. Verfahren zur Herstellung eines Kompressionstextilartikels (1, 12), umfassend den folgenden Schritt:
a) durchgehendes Stricken eines ersten röhrenförmigen Kompressionselements (2, 14), eines röhrenförmigen Zwischenelements (6, 16), dann eines zweiten röhrenförmigen Kompressionselements (3, 15), wobei das erste (2, 14) und das zweite (3, 15) Element dazu bestimmt sind, beim Überziehen des Artikels (1, 12) übereinander gelegt zu werden,
**dadurch gekennzeichnet, daß** es den folgenden Schritt umfaßt:
b) Verbinden des ersten (2, 14) und zweiten (3, 15) Elements entlang einem kreisförmigen Verbindungsbereich (9, 13) entweder durch Übertragen von Maschen beim Stricken im Laufe des Schrittes a) oder nach Schritt a) durch Hineinschieben des zweiten Elements (3, 15) in das erste Element (2, 14) und durch Ausbilden des Verbindungsbereiches (9, 13) durch Nähen, Ultraschall- oder Hochfrequenzschweißen oder Kleben, so daß das Zwischenelement (6, 16) sich von dem kreisförmigen Bereich (9, 13) aus erstreckt und daß sein vorderes (7) und sein hinteres (8) Ende mit dem Verbindungsbereich (9, 13) verbunden sind, um einen Umschlag (10, 17) zu bilden.

11. Herstellungsverfahren nach Anspruch 10, **dadurch gekennzeichnet, daß** es einen Schritt c) umfaßt, im Laufe dessen der Teil (18) des Umschlags (17), welcher von dem Verbindungsbereich (13) abgewandt ist, verschlossen, insbesondere zugenäht wird, um nach dem Überziehen das distale Ende des Artikels, insbesondere die Fußspitze zu bilden.

12. Herstellungsverfahren nach dem einen oder anderen der Ansprüche 10 und 11, **dadurch gekennzeichnet, daß** der Schritt des durchgehenden Strickens auf einer viersystemigen Rundstrickmaschine, vorzugsweise in Teilung 20 vollzogen wird.
